Europäisches Patentamt

(19) European Patent Office

Office européen des brevets

(11) Veröffentlichungsnummer: **0 029 495**

**A1**

(12) # EUROPÄISCHE PATENTANMELDUNG

(21) Anmeldenummer: 80105812.4

(22) Anmeldetag: 25.09.80

(51) Int. Cl.³: **C 07 C 120/02**
**C 07 C 121/417**

(30) Priorität: 24.11.79 DE 2947475

(43) Veröffentlichungstag der Anmeldung:
03.06.81 Patentblatt 81/22

(84) Benannte Vertragsstaaten:
BE CH FR GB IT LI NL SE

(71) Anmelder: Degussa Aktiengesellschaft
Degussa AG Fachbereich Patente Rodenbacher
Chaussee 4 Postfach 1345
D-6450 Hanau 1 (Stadtteil Wolfgang)(DE)

(72) Erfinder: Kleemann, Axel Dr. Dipl.-Chem.
Greifenhagenstrasse 9
D-6450 Hanau 9(DE)

(72) Erfinder: Leuchtenberger, Wolfgang, Dr. Dipl.-Chem.
Geschwister-Scholl-Strasse 1
D-6454 Bruchköbel(DE)

(72) Erfinder: Martens, Jürgen, Dr. Dipl.-Chem.
Hochstrasse 5
D-8755 Alzenau(DE)

(72) Erfinder: Weigel, Horst
Odenwaldstrasse 56
D-6458 Rodenbach(DE)

(54) Verfahren zur Herstellung von 3-Cyanopropionsäureamid.

(57) Verfahren zur Herstellung von 3-Cyanopropionsäureamid 3-Cyanopropionsäureamid wird hergestellt durch Anlagerung von Blausäure an Acrylsäureamid in Gegenwart eines aprotischen organischen Lösungsmittels und eines Alkalimetallcyanids bei einer Temperatur zwischen 20 und 150°C. 3-Cyanopropionsäureamid ist ein wichtiges Zwischenprodukt für die Herstellung von Pyrrolidon oder 4-Aminobuttersäureamid.

EP 0 029 495 A1

0029495

- 1 -

Degussa Aktiengesellschaft
Weissfrauenstrasse 9

6000 Frankfurt/Main

Verfahren zur Herstellung von 3-Cyanopropionsäureamid

3-Cyanopropionsäureamid ist ein wichtiges Zwischenprodukt für die Herstellung von Pyrrolidon oder
des als Pharmazeutikum interessanten 4-Aminobuttersäureamids.

Bekannte Verfahren zur Herstellung von 3-Cyanopropionsäureamid gehen aus von Bernsteinsäuredinitril, das
partiell verseift wird (vgl. GB-PS 782 258 oder
DE-PS 1 132 913). Ist schon Bernsteinsäuredinitril
vergleichsweise schwierig herzustellen, so liefert
seine partielle Hydrolyse obendrein auch nur unbefriedigende Einsatzausbeuten an 3-Cyanopropionsäureamid.

Gegenstand der Erfindung ist nun ein Verfahren zur
Herstellung von 3-Cyanopropionsäureamid, welches

dadurch gekennzeichnet ist, dass man Blausäure in Gegenwart eines aprotischen organischen Lösungsmittels und eines Alkalimetallcyanids bei einer Temperatur zwischen 20 und 150°C an Acrylsäureamid anlagert.

Blausäure (Cyanwasserstoff) wie Acrylsäureamid sind leicht zugängliche Materialien. Die Anlagerung der Blausäure an das Acrylsäureamid verläuft überraschenderweise sehr glatt und führt in hoher Regioselektivität und sehr guten Ausbeuten zum 3-Cyanopropionsäureamid. Eine Polymerisation des Acrylsäureamids erfolgt allenfalls in sehr untergeordnetem Masse.

Die Blausäure wird zweckmässigerweise in stöchiometrischer Menge zu dem Acrylsäureamid eingesetzt, kann jedoch auch in einem geringen Überschuss angewandt werden.

Die Umsetzung erfolgt in Gegenwart eines aprotischen organischen Lösungsmittels. Besonders bevorzugte Lösungsmittel sind Dimethylformamid und Dimethylsulfoxid. Weitere geeignete Lösungsmittel sind beispielsweise Diethylformamid, Dimethylacetamid, N-Methylpyrrolidon, Tetrahydrofuran, Tetramethylensulfon (Sulfolan) und Tetramethylharnstoff, sowie Homologe der genannten Verbindungen mit bis zu insgesamt 8 Kohlenstoffatomen im Molekül. Die Lösungsmittelmenge ist nicht kritisch, sollte aber zweckmässigerweise so bemessen sein, dass bei der gewählten Reaktionstemperatur das eingesetzte Acrylsäureamid wie auch das gebildete 3-Cyanopropionsäureamid in gelöster Form vorliegen.

Das erfindungsgemässe Verfahren erfordert ferner die Anwesenheit eines Alkalimetallcyanids als Katalysator. Bevorzugt werden Natrium- oder Kaliumcyanid verwendet. Auch die Katalysatormenge ist nicht eigentlich kritisch. Zur Erzielung kurzer Reaktionszeiten ist es jedoch vorteilhaft, den Katalysator in Mengen von 0,1 bis 50 , vorzugsweise von 10 bis 20 , Gewichtsprozent, bezogen auf die eingesetzte Menge an Blausäure, einzusetzen.

Die Umsetzung erfolgt bei einer Temperatur zwischen 20 und 150°C, vorzugsweise zwischen 70 und 120°C. Der Druck hat keinen feststellbaren Einfluss auf die Reaktionsgeschwindigkeit und die Zusammensetzung des Reaktionsgemisches nach beendeter Umsetzung.

Das erfindungsgemässe Verfahren kann so durchgeführt werden, dass man eine Suspension des Katalysators in einem Teil des Lösungsmittels vorlegt und eine Lösung des Acrylsäureamids und der Blausäure im restlichen Teil des Lösungsmittels zudosiert. Ebensogut kann man aber auch eine Lösung des Acrylsäureamids, in welcher der Katalysator suspendiert ist, vorlegen und die Blausäure einleiten. Zur Gewinnung des gebildeten 3-Cyanopropionsäureamids wird nach beendeter Umsetzung die Lösung eingeengt und mit einem Fällungsmittel, beispielsweise Toluol, versetzt, worauf das 3-Cyanopropionsäureamid auskristallisiert.

Die Erfindung wird durch die nachstehenden Beispiele näher erläutert:

Beispiel 1:

Zu einer Suspension von 2 g Kaliumcyanid in 75 ml Dimethylformamid wird bei Temperaturen von 90 bis 110°C eine Lösung von 71,08 g (1 Mol) Acrylsäureamid und 27 g (1 Mol) Blausäure in weiteren 75 ml Dimethylformamid langsam zugetropft. Nach einer Nachreaktionszeit von etwa 5 Minuten werden im Wasserstrahlvakuum 100 ml Dimethylformamid abdestilliert. Der noch warme Destillationsrückstand wird mit 150 ml Toluol verrührt. Dabei kristallisiert das gebildete 3-Cyanopropionsäureamid aus. Nach dem Abfiltrieren und Trocknen bei 60 bis 70°C wird dessen Menge zu 89,1 g (91 % der Theorie) bestimmt. Schmelzpunkt: 90 bis 93°C, nach dem Umkristallisieren aus Essigsäureethylester: 93 bis 95°C.

$C_4H_6N_2O$ (98,11)

|  | C | H | N |
|---|---|---|---|
| berechnet: | 48,97 % | 6,16 % | 28,56 % |
| gefunden: | 48,97 % | 6,06 % | 28,32 %. |

Beispiel 2:

Das Beispiel 1 wird wiederholt mit dem einzigen Unterschied, dass anstelle des Kaliumcyanids 2 g Natriumcyanid verwendet werden. Ausbeute: 89 % der Theorie. Schmelzpunkt: 90 bis 93°C (umkristallisiert aus Isopropanol).

Beispiel 3:

Das Beispiel 1 wird wiederholt mit dem einzigen Unterschied, dass anstelle des Dimethylformamids die gleiche Volumenmenge an Dimethylsulfoxid verwendet wird. Ausbeute: 88 % der Theorie. Schmelzpunkt: 89 bis 92°C (umkristallisiert aus n-Butanol).

Beispiel 4:

Das Beispiel 1 wird wiederholt mit dem Unterschied, dass anstelle des Kaliumcyanids 2 g Natriumcyanid und anstelle des Dimethylformamids die gleiche Volumenmenge an Dimethylsulfoxid verwendet werden. Ausbeute: 89 % der Theorie. Schmelzpunkt: 89 bis 92°C.

Beispiel 5:

In einer Lösung von 71,08 g (1 Mol) Acrylsäureamid in 100 ml Dimethylformamid werden 2 g Kaliumcyanid suspendiert. Dann werden bei 40 bis 50°C 27 g (1 Mol) Blausäure eingeleitet. Nach beendeter Blausäureeinleitung wird das Reaktionsgemisch eine Stunde lang auf einer Temperatur zwischen 70 und 100°C gehalten. Das Dimethylformamid wird zur Hälfte abdestilliert und der Rückstand mit 150 ml Toluol verrührt. Man erhält 91,1 g 3-Cyanopropionsäureamid (93 % der Theorie) mit einem Schmelzpunkt von 93 bis 95°C.

Beispiel 6:

Das Beispiel 5 wird wiederholt mit dem einzigen Unterschied, dass anstelle des Kaliumcyanids 2 g Natriumcyanid verwendet werden. Ausbeute: 89 % der Theorie. Schmelzpunkt: 90 bis 93°C.

23. September 1980
PAT/Dr.Sib-El

Degussa Aktiengesellschaft
Weissfrauenstrasse 9

6000 Frankfurt/Main

Verfahren zur Herstellung von 3-Cyanpropionsäureamid

Patentansprüche:

1. Verfahren zur Herstellung von 3-Cyanopropionsäureamid, dadurch gekennzeichnet, dass man Blausäure in Gegenwart eines aprotischen organischen Lösungsmittels und eines Alkalimetallcyanids bei einer Temperatur zwischen 20 und 150°C an Acrylsäureamid anlagert.

2. Verfahren nach Anspruch 1, dadurch gekennzeichnet, dass man als aprotisches organisches Lösungsmittel Dimethylformamid oder Dimethylsulfoxid einsetzt.

3. Verfahren nach Anspruch 1 oder 2, dadurch gekennzeichnet, dass man als Alkalimetallcyanid Natrium- oder Kaliumcyanid einsetzt.

0029495

4. Verfahren nach einem der Ansprüche 1 bis 3, dadurch gekennzeichnet, dass man die Umsetzung bei einer Temperatur zwischen 70 und 120$^{o}$C vornimmt.

Europäisches
Patentamt

**EUROPÄISCHER RECHERCHENBERICHT**

Nummer der Anmeldung

EP 80 10 5812.4

| | EINSCHLÄGIGE DOKUMENTE | | | KLASSIFIKATION DER ANMELDUNG (Int. Cl.³) |
|---|---|---|---|---|
| Kategorie | Kennzeichnung des Dokuments mit Angabe, soweit erforderlich, der maßgeblichen Teile | | betrifft Anspruch | |
| | DE - B - 1 287 057 (DEUTSCHE GOLD- UND SILBER-SCHEIDEANSTALT) * Beschreibung und Beispiel 7 * | | 1-4 | C 07 C 120/02 C 07 C 121/417 |
| & | US - A - 3 644 467 | | | |
| & | US - A - 3 644 468 | | | |
| | -- | | | |
| D,A | DE - C - 1 132 913 (KNAPSACK-GRIESHEIM) * ganzes Dokument * | | | |
| | -- | | | |
| D,A | GB - A - 782 258 (INVENTA) * ganzes Dokument * | | | **RECHERCHIERTE SACHGEBIETE (Int. Cl.³)** |
| | ---- | | | C 01 C 3/10 |
| | | | | C 07 C 120/00 |
| | | | | C 07 C 120/02 |
| | | | | C 07 C 121/00 |
| | | | | C 07 C 121/40 |
| | | | | C 07 C 129/417 |

**KATEGORIE DER GENANNTEN DOKUMENTE**

X: von besonderer Bedeutung

A: technologischer Hintergrund

O: nichtschriftliche Offenbarung

P: Zwischenliteratur

T: der Erfindung zugrunde liegende Theorien oder Grundsätze

E: kollidierende Anmeldung

D: in der Anmeldung angeführtes Dokument

L: aus andern Gründen angeführtes Dokument

&: Mitglied der gleichen Patentfamilie, übereinstimmendes Dokument

| X | Der vorliegende Recherchenbericht wurde für alle Patentansprüche erstellt. |
|---|---|

| Recherchenort | Abschlußdatum der Recherche | Prüfer |
|---|---|---|
| Berlin | 03-03-1981 | BREW |

EPA form 1503.1 06.78